# FASCICULE DE BREVET EUROPEEN

(11) **EP 0 518 772 B1**
(45) Date de publication et mention de la délivrance du brevet: **02.11.1994**
(21) Numéro de dépôt: 92401626.4
(22) Date de dépôt: 12.06.1992
(51) Int. Cl.: A61K 7/42, A61K 7/00

(54) **Composition cosmétique filtrante contenant un mélange d'acide benzène 1,4-di(3-méthylidène-10-camphosulfonique) partiellement ou totalement neutralisé et de nanopigments d'oxydes métalliques**
Filterndes kosmetisches Mittel enthaltend eine Mischung aus teilweise oder vollständig neutralisierten Benzol 1,4-di(3-Methyliden-10-Kamphosulfonsäure) und Metalloxid-Nanopigmenten
Sunscreening cosmetic composition containing a blend of partly or completely neutralised benzene 1,4-di(3-methylidene-10-camphosulfonic acid) and metallic oxids nanopigments

(30) Priorité: 13.06.1991 FR 9107255
(43) Date de publication de la demande: 16.12.1992
(73) Titulaire: L'OREAL, 75008 Paris (FR)
(72) Inventeur: Eteve, Martine, F-75013 Paris (FR); Hansenne, Isabelle, F-75017 Paris (FR)
(74) Mandataire: Casalonga, Axel

(56) Documents cités:
- EP-A- 0 303 995
- EP-A- 0 370 867
- DE-A- 3 824 999

## Description

La présente invention a pour objet une composition cosmétique filtrante renfermant l'acide benzène 1,4-di(3-méthylidène-10-camphosulphonique) partiellement ou totalement neutralisé et au moins un nanopigment d'oxyde métallique, ainsi que son utilisation pour la protection de l'épiderme humain et des cheveux et comme produit de maquillage.

L'acide benzène 1,4-di(3-méthylidène-10-camphosulfonique) et ses sels, décrits dans les brevets français de la demanderesse n° 2 528 420 et 2 639 347, sont des filtres dits à large bande qui absorbent les rayons ultraviolets de longueurs d'onde comprises entre 280 et 400 nm, avec des maxima d'absorption compris entre 320 et 400 nm, se situant notamment aux alentours de 345 nm. Cependant, les compositions cosmétiques renfermant de tels filtres ont l'inconvénient de laisser, sur la peau et les cheveux, un toucher relativement collant.

De plus, l'efficacité des compositions cosmétiques renfermant ces filtres UV à large spectre, exprimée par le facteur de protection solaire que l'on convient d'appeler "indice de protection ou IP", est bonne, mais s'avère encore insuffisante pour des peaux très sensibles ou continuellement exposées au rayonnement solaire.

L'indice de protection ou IP peut s'exprimer par le rapport du temps d'irradiation nécessaire pour atteindre le seuil érythématogène avec le filtre UV au temps d'irradiation nécessaire pour atteindre le seuil érythématogène sans filtre UV.

On connaît par ailleurs, l'utilisation d'oxydes métalliques classiques de granulométrie comprise entre 100 et 700 nm tels que l'oxyde de titane, dans les produits de maquillage où celui-ci est utilisé en tant que pigment blanc opacifiant en association avec des pigments colorés. Ces composés sont particulièrement intéressants du fait de leurs propriétés de diffusion et de réflexion du rayonnement ultraviolet, qui permettent de protéger l'épiderme humain contre les rayons ultraviolets. Cependant, lorsque l'on augmente la concentration en oxyde de titane dans une composition cosmétique afin d'accroître la protection contre les rayons ultraviolets, on obtient un produit cosmétique difficile à étaler sur la peau, opaque et laissant subsister sur la peau, après application, une pellicule blanchâtre peu appréciée des utilisateurs.

On a donc essayé de réduire la granulométrie des pigments d'oxydes métalliques. Toutefois, on s'est aperçu que l'exposition à la lumière de pigments d'oxydes métalliques de granulométrie inférieure à 100 nm, appelés "nanopigments", peut provoquer une réaction photoinduite préjudiciable à la stabilité des compositions cosmétiques, en particulier celles qui contiennent des lipides.

Selon l'invention, on a découvert d'une façon surprenante que l'association de nanopignents d'oxydes métalliques de granulométrie inférieure à 100 nm avec l'acide benzène 1,4-di (3 -méthylidène-10-camphosulphonique) partiellement ou totalement neutralisé, dans une composition cosmétique, permet d'une part, d'améliorer les propriétés cosmétiques de cette dernière et notamment de diminuer l'effet collant du filtre et le blanchiment subsistant sur la peau après application et d'autre part, de diminuer ou d'inhiber la réaction photoinduite des nanopigments d'oxydes métalliques. Les compositions cosmétiques ainsi obtenues présentent également un indice de protection élevé dans l'UV-B et, par leur forte absorption dans l'UV-A, permettent de prévenir l'apparition des photodermatoses, dont les lucites estivales bénignes ou polymorphes. Elles présentent par ailleurs une bonne rémanence à l'eau, c'est-à-dire une bonne stabilité de l'indice de protection au cours du temps, notamment après la douche ou la baignade.

La présente invention a donc pour objet une composition cosmétique filtrante comprenant, en mélange, l'acide benzène 1,4-di(3-méthylidène-10-camphosulfonique) partiellement ou totalement neutralisé de formule (I) ci-dessous, et au moins un nanopigment d'oxydes métalliques dans un support cosmétiquement acceptable.

L'acide benzène 1,4-di(3-méthylidène-10-camphosulfonique), partiellement ou totalement neutralisé, a pour formule : 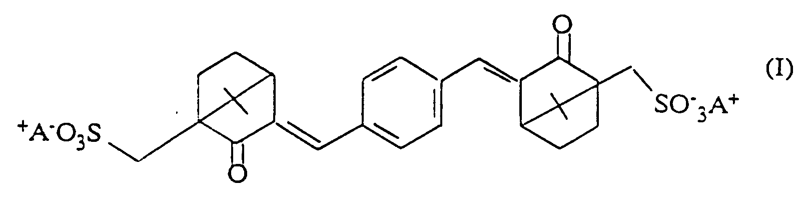
dans laquelle A désigne un atome d'hydrogène, un métal alcalin, un groupement NH(R)₃⁺, les radicaux R étant identiques ou différents et désignant un atome d'hydrogène ou un radical alkyle ou hydroxyalkyle en C₁-C₄, ou encore un groupement Mⁿ⁺ /n où Mⁿ⁺ est un cation métallique polyvalent dans lequel n est égal à 2 ou 3 ou 4, Mⁿ⁺ désignant de préférence Ca²⁺, Zn²⁺, Mg²⁺, Ba²⁺, Al³⁺ ou Zr⁴⁺.

Il est bien entendu que le composé de formule (I) peut donner lieu à l'isomère "cis-trans" autour d'une ou plusieurs double(s) liaison(s) et que tous les isomères font partie de l'invention.

Dans la présente demande, on entendra par "nanopigment" un pigment de diamètre moyen inférieur à 100 nm et de préférence compris entre 5 et 50 nm.

Les oxydes métalliques sont choisis parmi les oxydes de titane, de zinc, de cérium, de zirconium ou leurs mélanges.

Les nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés:
- de silice tel que le produit "SUNVEIL" de la société IKEDA,
- de silice et d'oxyde de fer tel que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et" UVT 14/4" de la société KEMIRA,
- d'alumine et de stéarate d'aluminium tels que le produit "MICROTITANIUM DIOXIDE MT 100 T" de la société TAYCA,
- d'alumine et de laurate d'aluminium tel que le produit "MICROTITANIUM DIOXIDE MT 100 S" de la société TAYCA,
- d'oxyde de fer et de stéarate de fer tel que le produit "MICROTITANIUM DIOXIDE MT 100 F" de la société TAYCA,
- d'oxyde de zinc et de stéarate de zinc tel que le produit "BR 351" de de la société TAYCA,
- de silice, d'alumine et de silicone tels que les produits "MICROTITANIUM DIOXIDE MT 600 SAS " et "MICROTITANIUM DIOXIDE MT 500 SAS" de la société TAYCA,
- de silice, d'alumine, de stéarate d'aluminium et de silicone tel que le produit "STT-30-DS" de la société TITAN KOGYO,
- d'alumine et de silicone tel que le produit "TIPAQUE TTO-55 (S)" de la société ISHIHARA,
- de triéthanolamine tel que le produit "STT-65-S " de la société TITAN KOGYO,
- d'acide stéarique tel que le produit "TIPAQUE TTO-55 (C)" de la société ISHIHARA,
- d'hexamétaphosphate de sodium tel que le produit "MICROTITANIUM DIOXIDE MT 150 W" de la société TAYCA.

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les oxydes de titane non enrobés sont par exemple vendus par la société TAYCA sous les dénominations commerciales "MICROTITANIUM DIOXIDE MT 500 B" ou "MICROTITANIUM DIOXIDE MT 600 B", par la société DEGUSSA sous la dénomination "P 25", par la société WACKHERR sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25" ou par la société IKEDA sous la dénomination "MZO-25".

L'oxyde de cérium non enrobé est vendu sous la dénomination "COLLOIDAL CERIUM OXIDE" par la société RHONE POULENC.

Selon l'invention, les nanopigments d'oxyde de titane, enrobés ou non enrobés, sont particulièrement préférés.

L'acide benzène 1,4-di(3-méthylidène-10-camphosulfonique), partiellement ou totalement neutralisé de formule (I) ci-dessus, est avantageusement présent dans la composition cosmétique selon l'invention à une concentration, calculée sur la base de l'acide, comprise entre 0,1 et 10% en poids et de préférence entre 0,25 et 5% en poids par rapport au poids total de la composition.

Les nanopigments d'oxydes métalliques sont avantageusement présents dans la composition cosmétique selon l'invention à une concentration comprise entre 0,5 et 10% en poids, et de préférence comprise entre 1 et 7% en poids, par rapport au poids total de la composition.

Le rapport en poids acide benzène 1,4-di(3-méthylidène-10-camphosulfonique)/nanopigment d'oxyde métallique est avantageusement compris entre 0,05 et 5, de préférence entre 0,07 et 3.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de dispersion vésiculaire, de crème, de lait, de poudre, de bâtonnet solide et éventuellement être conditionnée en aérosol et se présenter sous forme de mousse ou de spray.

Elle peut contenir les adjuvants cosmétiques habituellement utilisés tels que des corps gras, des solvants organiques, des silicones, des épaississants, des adoucissants, des filtres solaires UV-A, UV-B, ou à bande large, des agents anti-mousses, des agents hydratants, des parfums, des conservateurs, des tensio-actifs, des charges, des séquestrants, des polymères anioniques, cationiques, non-ioniques, amphotères, ou leurs mélanges, des propulseurs, des agents alcalinisants ou acidifiants, des colorants, des pigments d'oxydes métalliques de granulométrie comprise entre 100 nm et 20 000 nm comme les oxydes de fer, ou tout autre ingrédient habituellement utilisé en cosmétique.

Parmi les solvants organiques, on peut citer les alcools et polyols inférieurs tels que l'éthanol, l'isopropanol, le propylène glycol, la glycérine et le sorbitol.

Les corps gras peuvent être constitués par une huile ou une cire ou leur mélange, les acides gras, les esters d'acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin, les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Les esters d'acides gras sont par exemple le myristate d'isopropyle, l'adipate d'isopropyle, le palmitate d'isopropyle, le palmitate d'octyle, les benzoates d'alcools gras en C₁₂-C₁₅ ("FINSOLV TN" de FINETEX), l'alcool myristique oxypropyléné à 3 moles d'oxyde de propylène ("WITCONOL APM" de WITCO), les triglycérides d'acides caprique et caprylique ("MIGLYOL 812" de HULS).

La composition cosmétique selon l'invention peut aussi contenir des épaississants qui peuvent être choisis parmi les polymères d'acide acrylique réticulés ou non, et particulièrement les acides polyacryliques réticulés par un agent polyfonctionnel tels que les produits vendus sous la dénomination "CARBOPOL" par la société GOODRICH, les dérivés de cellulose tels que la méthylcellulose, l'hydroxyéthylcellulose, l'hydroxypropylmétlylcellulose, les sels de sodium de la carboxyméthylcellulose, les mélanges d'alcool cétylstéarylique et d'alcool cétylstéarylique oxyéthyléné à 33 moles d'oxyde d'éthylène.

On peut également utiliser les produits résultant de l'interaction ionique d'un polymère cationique constitué par un copolymère de cellulose ou d'un dérivé de cellulose greffés par un sel de monomère hydroxoluble d'ammonium quaternaire et d'un polymère anionique carboxylique tels que décrits dans le brevet français FR-2 598 611. On utilise de préférence le produit d'interaction ionique d'un copolymère d'hydroxyéthylcellulose greffé par voie radicalaire par du chlorure de diallyldiméthylammonium tel que le polymère commercialisé sous la dénomination "CELQUAT L 200" par la société NATIONAL STARCH avec, soit des copolymères d'éthylène et d'anhydride maléique tels que les produits vendus sous la dénomination "EMA 31" par la société MONSANTO, soit des copolymères 50/50 d'acide méthacrylique et de méthacrylate de méthyle.

Un autre produit de ce type utilisable est le produit résultant de l'interaction ionique du copolymère d'hydroxyéthylcellulose greffé par voie radicalaire par du chlorure de diallyldiméthylammonium avec un polymère anionique carboxylique réticulé tel que les copolymères de l'acide méthacrylique et de l'acrylate d'éthyle réticulés vendus sous la dénomination "VISCOATEX" 538, 46, ou 50 par la société COATEX.

Lorsque la composition cosmétique selon l'invention est utilisée pour la protection de l'épiderme humain contre les rayons UV, comme composition antisolaire ou comme composition permettant de prévenir les photodermatoses et les lucites, elle peut se présenter sous forme de suspension ou de dispersion dans des solvants ou des corps gras, ou encore sous forme d'émulsion telle qu'une crène ou un lait, sous forme de pommade, de gel, de bâtonnet solide ou de mousse aérosol. Les émulsions peuvent contenir en outre des agents tensio-actifs anioniques, non-ioniques, cationiques ou amphotères.

Elle peut se présenter aussi sous forme de dispersion vésiculaire de lipides amphiphiles ioniques ou non-ioniques, préparée selon des procédés connus. On peut, par exemple, faire gonfler les lipides dans une solution aqueuse pour former des sphérules dispersées dans le milieu aqueux comme décrit dans l'article BANGHAN, STANDISH & WATKINS, J. mol. Biol., 13, 238 (1965) ou dans les brevets FR-2 315 991 et 2 416 008 de la demanderesse.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffants ou traitants, de lotion ou gel pour le brushing ou la mise en plis, de laque pour cheveux, de composition de permanente ou de défrisage, de coloration ou décoloration des cheveux.

Lorsque la composition est utilisée comme produit de maquillage des cils, des sourcils, de la peau ou des cheveux, tel que crème de traitement de l'épiderme, fond de teint, bâton de rouge à lèvres, fard à paupières, fard à joues, ligneur encore appelé "eye-liner", mascara ou gel colorant, elle peut se présenter sous forme solide ou pâteuse, anhydre ou aqueuse, comme des émulsions huile-dans-eau ou eau-dans-huile, des suspensions, ou encore des gels.

L'invention a également pour objet un procédé de protection de l'épiderme humain et des cheveux contre le rayonnement ultraviolet ainsi qu'un procédé de maquillage consistant à appliquer sur la peau ou les cheveux une quantité efficace de la composition cosmétique ci-dessus.

L'invention a aussi pour objet l'utilisation de la composition définie ci-dessus dans le traitement préventif des photodermatoses dont les lucites estivales bénignes ou polymorphes.

Un autre objet de l'invention concerne l'utilisation de l'acide benzène 1,4-di(3-méthylidène-10 camphosulfonique), partiellement ou totalement neutralisé, pour diminuer ou inhiber la réaction photoinduite des nanopignents d'oxydes nétalliques exposés à la lumière, ces oxydes métalliques étant choisis parmi les oxydes de titane, de zinc, de cérium, de zirconium ou leurs mélanges ayant un diamètre moyen inférieur à 100 nm et de préférence compris entre 5 et 50 nm.

L'invention sera mieux illustrée par les exemples ci-après.

### EXEMPLE 1

On prépare une émulsion antisolaire huile-dans-eau de composition suivante:

| | |
|---|---|
| - Acide benzène 1,4-di(3-méthylidène-10-camphosulfonique) | 5,0 g |
| - Oxyde de titane enrobé d'alumine et de stéarate d'aluminium vendu sous la dénomination "Micro TITANIUM DIOXIDE MT 100T" par la société TAYCA | 5,0 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| - Benzoate d'alcools en C₁₂-C₁₅ vendu sous la dénomination "FINSOLV TN" par la société FINETEX | 15,0 g |
| - Alcool cétylique | 1,5 g |
| - Polydiméthylsiloxane | 1,5 g |
| - Glycérine | 20,0 g |
| - Triéthanolamine qs pH7 | |
| - Conservateurs, parfum qs | |
| - Eau qsp | 100 g |

### EXEMPLE 2

On prépare une émulsion antisolaire huile-dans-eau de composition suivante :

| | |
|---|---|
| - Acide benzène 1,4-di(3-méthylidène-10-camphosulfonique) | 1,0 g |
| - Oxyde de titane enrobé d'alumine et de stéarate d'aluminium vendu sous la dénomination "Micro TITANIUM DIOXIDE MT 100T" par la société TAYCA | 6,0 g |
| - 3-(4-méthylbenzylidène) camphre vendu sous la dénomination "EUSOLEX 6300" par la société MERCK | 3,0 g |
| - 4-tert. butyl-4'-méthoxy-dibenzoylméthane vendu sous la dénomination "PARSOL 1789" par la société GIVAUDAN | 1,0 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| - Benzoate d'alcools en C₁₂C₁₅ vendu sous la dénomination "FINSOLV TN" par la société FINETEX | 15,0 g |
| - Alcool cétylique | 1,5 g |
| - Polydiméthylsiloxane | 1,5 g |
| - Glycérine | 20,0 g |
| - Triéthanolamine qs pH7 | |
| - Conservateur, parfum qs | |
| - Eau qsp | 100 g |

Cette composition peut être appliquée pour prévenir les lucites bénignes ou polymorphes.

### EXEMPLE 3

On prépare une émulsion antisolaire huile-dans-eau de composition suivante :

| | |
|---|---|
| - Acide benzène 1,4-di(3-méthylidène-10-camphosulfonique) | 2,0 g |
| - Oxyde de titane enrobé d'alumine et de stéarate d'aluminium vendu sous la dénomination "Micro TITANIUM DIOXIDE MT 100T" par la société TAYCA | 4,0 g |
| - α-cyano-β,β-diphénylacrylate de 2-éthylhexyle vendu sous la dénomination "UVINUL N 539" par la société BASF | 6,0 g |
| - 4-tert.butyl-4'-méthoxy-dibenzoylméthane vendu sous la dénomination "PARSOL 1789" par la société GIVAUDAN | 2,0 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| - Benzoate d'alcools en C₁₂-C₁₅ vendu sous la dénomination "FINSOLV TN" par la société FINETEX | 15,0 g |
| - Alcool cétylique | 1,5 g |
| - Polydiméthylsiloxane | 1,5 g |
| - Glycérine | 20,0 g |
| - Triéthanolamine qs pH7 | |
| - Conservateurs, parfum qs | |
| - Eau qsp | 100 g |

### EXEMPLE 4

On prépare une émulsion antisolaire huile-dans-l'eau de composition suivante :

| | |
|---|---|
| - Acide benzène 1,4-di(3-méthylidène-10-campho sulfonique) | 5,0 g |
| - Oxyde de zinc vendu sous la dénomination "ULTRA FINE ZINC POWDER" par la société SUMITOMO | 5,0 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| - Benzoate d'alcools en C₁₂-C₁₅ vendu sous la dénomination "FINSOLV TN" par la société FINETEX | 15,0 g |
| - Alcool cétylique | 1,5 g |
| - Polydiméthylsiloxane | 1,5 g |
| - Glycérine | 20,0 g |
| - Triéthanolamine qs pH 7 | |
| - Conservateurs, parfum qs | |
| - Eau qsp | 100g |

### EXEMPLE 5

On prépare une émulsion antisolaire huile-dans-eau de composition suivante :

| | |
|---|---|
| - Sel d'aluminium de l'acide benzène 1,4-di(3-méthylidène-10-campho-sulfonique) | 5,0 g |
| - Oxyde de titane enrobé d'alumine et de stéarate d'aluminium vendu sous la dénomination "Micro TITANIUM DIOXIDE MT 100T" par la société TAYCA | 5,0 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| - Benzoate d'alcools en C₁₂-C₁₅ vendu sous la dénomination "FINSOLV TN" par la société FINETEX | 15,0 g |
| - Alcool cétylique | 1,5 g |
| - Polydiméthylsiloxane | 1,5 g |
| - Glycérine | 20,0 g |
| - Triéthanolamine qs pH7 | |
| - Conservateurs, parfum qs | |
| - Eau qsp | 100 g |

### EXEMPLE 6

On prépare un après-shampooing à rincer de composition suivante :

| | |
|---|---|
| - Acide benzène 1,4-di(3-méthylidène-10-campho-sulfonique) | 2,0 g |
| - Oxyde de cérium (diamètre moyen 12 nanomètres) en suspension aqueuse à 20% de MA vendu par la société RHONE POULENC sous la dénomination "COLLOIDAL CERIUM OXIDE" | 3,0 g MA |
| - Alcool cétylstéarylique polyoxyéthyléné à 33 moles d'oxyde d'éthylène | 1,0 g |
| - Alcool cétylstéarylique (C₁₆-C₁₈/50-50) | 2,0 g |
| - Alcool cétylstéarylique (C₁₆-C₁₈/30-70) | 45 g |
| - 2-octyldodécanol | 0,8 g |
| - Glycérine | 0,8 g |
| - Chlorure de stéaryl diméthyl benzyl ammonium | 2,0 g |
| - Hydrolysat de protéine (PM=2500) portant des groupements ammonium quaternaire comportant des groupements alkyle en C₁₈ vendu sous la dénomination "CROQUATS" à 43,7% de MA par la société CRODA | 0,3 g MA |
| - Triéthanolamine qs pH 6,5 | |
| - Conservateur, parfum qs | |
| - Eau qsp | 100 g |

### EXEMPLE 7

On prépare une crème de soin pour le visage de composition suivante :
Dans une première étape, on fait fondre, en agitant doucement à une température de 90°C-95°C, un mélange de 3,8 g de lipide non ionique de formule : 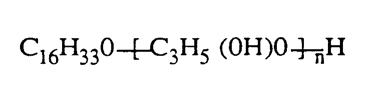
formule où n est une valeur statistique moyenne égale à 3 et où - C₃H₅ (0H)0 - est représenté par les structures suivantes prises en mélange ou séparément : 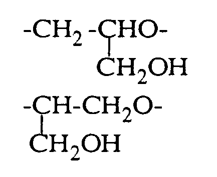
avec 3,8 g de cholestérol et 0,4 g du sel monosodique du glutamate de formule : 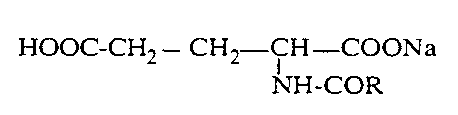
dans laquelle R est un mélange de radicaux alkényle et/ou alkyle hydrogénés en C₁₄-C₂₂ dérivé des acides gras du suif, vendu sous la dénomination commerciale "ACYLGLUTAMATE HS 11" par la société AJINOMOTO.

On introduit dans le mélange fondu, 16 g d'eau portée à 90°C contenant un conservateur et l'on mélange pendant environ 6 à 8 minutes au moyen d'un agitateur Ultra Turrax. . A la phase ainsi obtenue, on ajoute 24 g d'eau à température ambiante, puis on homogénéise le mélange à l'Ultra Turrax pendant encore 6 à 8 minutes avant de laisser revenir le mélange à la température ambiante.

Dans une seconde étape, on additionne les composés suivants :

| | |
|---|---|
| - Acide benzène 1,4-di(3-méthylidène-10-campho-sulfonique) | 1,0 g |
| - Oxyde de cérium (diamètre moyen 12 nanomètres) en suspension aqueuse à 20% de MA vendu par la société RHONE POULENC sous la dénomination "COLLOIDAL CERIUM OXIDE" | 2,0 g MA |
| - Glycérine | 3,0 g |
| - Huile de vaseline | 10,0 g |
| - Conservateur, parfum qs | |

On ajoute ensuite un gel aqueux homogène constitué de 0,4 g d'acide polyacrylique réticulé vendu sous la dénomination "CARBOPOL 940" par la société GOODRICH et de 30 g d'eau renfermant un conservateur puis on complète à 100 g avec de l'eau.

Le pH est ajusté à 6,5 avec de la triéthanolamine.

### EXEMPLE 8

On prépare une crème teintée de composition suivante :

| | |
|---|---|
| - Mélange de mono- et distéarate de glycérol non autoémulsionnable | 3,5 g |
| - Isostéarate de glycérol | 1,8 g |
| - Mélange d'huile minérale et d'alcool de lanoline vendu sous la dénomination "AMERCHOL L-101" par la société AMERCHOL | 3,1 g |
| - Palmitate d'isopropyle | 7,6 g |
| - Palmitate d'octyle | 7,0 g |
| - Ultramarine violet | 0,75 g |
| - Dioxyde de titane de granulométrie 200-300 nm | 3,0 g |
| - Oxyde de fer jaune | 1,0 g |
| - Oxyde de fer rouge | 0,6 g |
| - Oxyde de fer noir | 0,08 g |
| - Conservateurs | 0,5 g |
| - Parfum | 0,3 g |
| - Silicate d'aluminium et de magnésium | 1,5 g |
| - Talc | 4,46 g |
| - Triéthanolamine | 1,2 g |
| - Gomme de cellulose | 0,05 g |
| - Gomme de xanthane | 0,15 g |
| - Cyclométhicone (dénomination "CTFA Dictionary" : diméthylpolysiloxane cyclique) | 7,5 g |
| - Propylène glycol | 3,0 g |
| - Glycérine | 2,0 g |
| - Acide stéarique | 2,5 g |
| - Dioxyde de titane de granulométrie 30-40 nm | 6,0 g |
| - Sel de zinc de l'acide benzène 1,4-di(3-méthylidène-10-campho-sulfonique) | 2,0 g |
| - Eau qsp | 100 g |

On chauffe séparément à 80°C la phase grasse contenant les huiles et l'acide stéarique et la phase aqueuse contenant la triéthanolamine.

Le mélange est émulsionné à 80°C et refroidi lentement. Pendant le refroidissement, sont ajoutés : le mélange de pigments préalablement broyé dans le propylèneglycol et le cyclométhicone.

### EXEMPLE 9

On prépare un fond de teint de composition suivante :

| | |
|---|---|
| - Triéthanolamine | 1,0 g |
| - Stéarate de polyéthylène glycol à 2 moles d'oxyde d'éthylène | 0,53 g |
| - Mélange de mono- et distéarate de glycérol non autoémulsionnable | 0,35 g |
| - Silicate d'aluminium et de magnésium | 1.5 g |
| - Oxyde de fer jaune | 0,9 g |
| - Oxyde de fer rouge | 0,5 g |
| - Oxyde de fer noir | 0,2 g |
| - Dioxyde de titane de granulométrie 200-300 nm | 5,4 g |
| - Dioxyde de titane de granulométrie 30-40 nm | 8,0 g |
| - Sel de triéthanolamine de l'acide benzène 1,4-di(3-méthylidène-10-campho-sulfonique) | 3,0 g |
| - Conservateurs | 0,5 g |
| - Mélange de polyéthylène glycol à 6 moles d'oxyde d'éthylène et de polyéthylène glycol à 32 moles d'oxyde d'éthylène vendu sous la dénomination "CARBAWAX 1450" par la société UNION CARBIDE | 9,0 g |
| - Gomme de cellulose | 0,02 g |
| - Polyéthylène | 9,3 g |
| - Cyclométhicone (dénomination "CTFA Dictionary" : diméthylpolysiloxane cyclique) | 14,0 g |
| - Propylène glycol | 6,0 g |
| - Glycérine | 3,0 g |
| - Acide stéarique | 2,2 g |
| - Eau qsp | 100 g |

La composition est préparée de façon analogue à l'exemple 8.

### EXEMPLE 10

On prépare un fond de teint de composition suivante :

| | |
|---|---|
| - Mélange de mono- et distéarate de glycérol non autoémulsionnable | 2,2 g |
| - Triglycérides d'acides caprylique- caprique | 15,0 g |
| - Oxyde de fer jaune | 0,6 g |
| - Oxydes de fer brun, jaune | 0,4 g |
| - Oxyde de fer noir | 0,2 g |
| - Dioxyde de titane de granulométrie 200 - 300 nm | 5,0 g |
| - p-hydroxybenzoate de méthyle | 0,1 g |
| - p-hydroxybenzoate de propyle | 0,1 g |
| - Conservateurs | 0,3 g |
| - Acide benzène 1,4-di(3-méthylidène-10-campho-sulfonique) | 4,0 g |
| - Silicate de magnésium et d'aluminium | 1,0 g |
| - Oxyde de titane vendu sous la dénomination "TIOVEIL AQ" par la société TIOXIDE | 10,0 g |
| - Triéthanolamine | 2,0 g |
| - Carboxyméthylcellulose de sodium | 0,16 g |
| - Sel d'aluminium du produit de réaction de l'anhydride octénylsuccinique avec l'amidon vendu sous la dénomination "DRYFLO" par la Société NATIONAL STARCH | 5,0 g |
| - Cyclopentadiméthylsiloxane | 10,0 g |
| - Propylène glycol | 2,0 g |
| - Glycérine | 3,0 g |
| - Lauroyl sarcosinate de sodium en solution dans l'eau à 30% MA vendu sous la dénomination "ORAMIX L 30" par la société SEPPIC | 0,6 g |
| - Acide stéarique | 2,2 g |
| - Eau qsp | 100 g |

## Revendications

1. Composition cosmétique filtrante caractérisée par le fait qu'elle comprend, dans un support cosmétiquement acceptable, au moins un nanopigment d'oxydes métalliques choisis parmi les oxydes de titane, de zinc, de cérium, de zirconium ou leurs mélanges, de diamètre moyen inférieur à 100 nm, en mélange avec l'acide benzène 1,4-di(3-méthylidène-10-camphosulfonique) partiellement ou totalement neutralisé de formule : 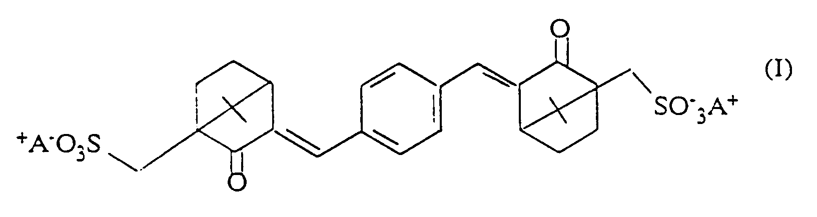 dans laquelle A désigne un atome d'hydrogène, un métal alcalin, un groupement NH(R)₃⁺, les radicaux R étant identiques ou différents et désignant un atome d'hydrogène ou un radical alkyle ou hydroxyalkyle en C₁-C₄ ou un groupement Mⁿ⁺ /n où Mⁿ⁺ est un cation métallique polyvalent dans lequel n est égal à 2 ou 3 ou 4.

2. Composition cosmétique selon la revendication 1, caractérisée par le fait que les nanopigments d'oxydes métalliques ont un diamètre moyen compris entre 5 et 50 nm.

3. Composition cosmétique selon la revendication 1 ou 2, caractérisée par le fait que l'oxyde métallique est l'oxyde de titane.

4. Composition cosmétique selon l'une quelconque des revendications 1 à 3, caractérisée par le fait que dans la formule (I), Mⁿ⁺ désigne Ca²⁺, Zn²⁺, Mg²⁺, Ba²⁺, Al³⁺ ou Zr⁴⁺.

5. Composition cosmétique selon l'une quelconque des revendications 1 à 4, caractérisée par le fait qu'elle comprend en mélange, un nanopigment d'oxyde de titane et le sel d'aluminium du composé de formule (I).

6. Composition cosmétique selon l'une quelconque des revendications 1 à 5, caractérisée par le fait que les nanopigments d'oxydes métalliques sont des pigments non enrobés.

7. Composition cosmétique selon l'une quelconque des revendications 1 à 6, caractérisée par le fait que les nanopigments d'oxydes métalliques sont des pigments enrobés ayant subi un ou plusieurs traitements de surface de nature chimique, électronique, mécanochimique ou mécanique avec des composés choisis parmi les aminoacides, la cire d'abeille, les acides gras, les alcools gras, les tensio-actifs anioniques, les lécithines, les sels de sodium, potassium, zinc, fer ou aluminium d'acides gras, l'hexamétaphosphate de sodium, les alcoxydes métalliques, le polyéthylène, les silicones, les protéines, les alcanolamines, les oxydes de silicium et les oxydes métalliques.

8. Composition cosmétique selon la revendication 7, caractérisée par le fait que les nanopigments d'oxydes métalliques enrobés sont des pigments d'oxydes de titane enrobés de silice, de silice et d'alumine, de silice et d'oxyde de fer, d'alumine et de silicone, d'alumine, d'alumine et de stéarate d'aluminium, d'alumine et de laurate d'aluminium, d'oxyde de fer et de stéarate de fer, d'oxyde de zinc et de stéarate de zinc, de silice et d'alumine et de silicone, de silice et d'alumine et de stéarate d'aluminium et de silicone, de triéthanolamine, d'acide stéarique ou d'hexamétaphosphate de sodium

9. Composition cosmétique selon la revendication 7, caractérisée par le fait que les nanopigments d'oxydes métalliques enrobés sont des mélanges de dioxyde de titane et de dioxyde de cérium enrobés de silice ou de dioxyde de titane et de dioxyde de zinc enrobés d'alumine, de silice et de silicone ou d'alumine, de silice et de glycérine.

10. Composition cosmétique selon l'une quelconque des revendications 1 à 9, caractérisée par le fait qu'elle contient 0,5 à 10%en poids, et de préférence 1 à 7% en poids, par rapport au poids total de la composition, d'au moins un nanopigment d'oxyde métallique.

11. Composition cosmétique selon l'une quelconque des revendications 1 à 10, caractérisée par le fait qu'elle contient 0,1 à 10% en poids, et de préférence 0,25 à 5% en poids, calculés sur la base de l'acide par rapport au poids total de la composition, d'acide benzène 1,4-di(3-méthylidène-10-camphosulfonique) partiellement ou totalement neutralisé de formule (I).

12. Composition cosmétique selon l'une quelconque des revendications 1 à 11, caractérisée par le fait que le rapport en poids acide benzène 1,4-di(3-méthylidène-10-camphosulfonique) partiellement ou totalement neutralisé /nanopigment d'oxyde métallique est compris entre 0,05 et 5, et de préférence entre 0,07 et 3.

13. Composition cosmétique selon l'une quelconque des revendications 1 à 12, caractérisée par le fait qu'elle se présente sous forme de lotion, lotion épaissie, gel, dispersion vésiculaire, crème, lait, poudre, bâtonnet solide, mousse ou spray.

14. Composition cosmétique selon la revendication 13, caractérisée par le fait qu'elle contient en outre des adjuvants cosmétiques choisis parmi les corps gras, les solvants organiques, les silicones, les épaississants, les adoucissants, les filtres solaires UV-A, UV-B ou à bande large, les agents antimousses, les agents hydratants, les parfums, les conservateurs, les tensio-actifs, les charges, les séquestrants, les polymères anioniques, cationiques, non-ioniques, amphotères ou leurs mélanges, les propulseurs, les agents alcalinisants ou acidifiants, les colorants et les pigments d'oxydes métalliques de granulométrie comprise entre 100 nm et 20 000 nm.

15. Composition cosmétique selon l'une quelconque des revendications 1 à 14, caractérisée par le fait qu'elle contient du 3-(4-méthylbenzylidène)camphre et du 4-tert.-butyl-4'-méthoxy-dibenzoylméthane, un nanopigment d'oxyde de titane et l'acide benzène 1,4-di(3-méthylidène-10-camphosulfonique) partiellement ou totalement neutralisé par la triéthanolamine.

16. Composition cosmétique selon l'une quelconque des revendications 1 à 15, constituant une composition protectrice de l'épiderme humain contre les rayons ultraviolets ou antisolaire,ou pour la prévention des photodermatoses, caractérisée par le fait qu'elle se présente sous forme de suspension ou de dispersion dans des solvants ou des corps gras, sous forme d'émulsion, sous forme de dispersion vésiculaire, sous forme de pommade, sous forme de gel, sous forme de bâtonnet solide ou de mousse aérosol.

17. Composition cosmétique selon l'une quelconque des revendications 1 à 14 utile pour la protection des cheveux contre les rayons ultraviolets, caractérisée par le fait qu'elle se présente sous forme de shampooing, de lotion, de gel ou composition à rincer, à appliquer avant, ou après shampooing, avant ou après coloration ou décoloration, avant, pendant ou après permanente ou défrisage, sous forme de lotion ou de gel coiffants ou traitants, de lotion ou gel pour le brushing ou la mise en plis, de laque pour cheveux, de composition de permanente ou de défrisage, de coloration ou décoloration des cheveux.

18. Composition cosmétique selon l'une quelconque des revendications 1 à 14 constituant un produit de maquillage des cils, de sourcils, de la peau ou des cheveux, caractérisée par le fait qu'elle se présente sous forme de crème de traitement de l'épiderme, fond de teint, bâton de rouge à lèvres, fard à paupières, fard à joues, ligneur encore appelé "eye-liner", mascara, gel colorant et se présente sous forme solide ou pâteuse, anhydre ou aqueuse.

19. Procédé de protection de l'épiderme humain et des cheveux contre le rayonnement ultraviolet, caractérisé par le fait qu'il consiste à appliquer sur la peau ou les cheveux une quantité efficace d'une composition cosmétique selon l'une quelconque des revendications 1 à 18.

20. Utilisation de la composition cosmétique selon l'une quelconque des revendications 1 à 16 pour prévenir l'apparition des photodermatoses.

21. Utilisation de l'acide 1,4-di(3-méthylidène-10-campho-sulfonique) partiellement ou totalement neutralisé pour diminuer ou inhiber la réaction photoinduite des nanopigments d'oxydes métalliques exposés à la lumière, ces oxydes métalliques étant choisis parmi les oxydes de titane, de zinc, de cérium, de zirconium ou leurs mélanges ayant un diamètre moyen inférieur à 100 nm et de préférence compris entre 5 et 50 nm.

## Claims

1. A screening cosmetic composition comprising, in a cosmetically acceptable carrier, at least one metal oxide nanopigment chosen from titanium, zinc, cerium or zirconium oxide or mixtures thereof, with a mean diameter of less than 100 nm, mixed with partially or completely neutralized 1,4-benzenedi(3-methylidene-10-camphosulfonic) acid of formula 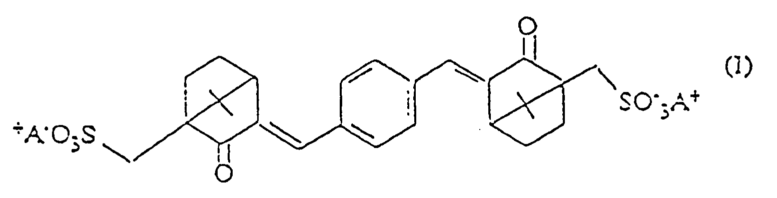 in which A denotes a hydrogen atom, an alkali metal or an NH(R)₃⁺ group, the R radicals being identical or different and denoting a hydrogen atom or a C₁-C₄ alkyl or hydroxyalkyl radical or an Mⁿ⁺/n group where Mⁿ⁺ is a polyvalent metal cation in which n is equal to 2 or 3 or 4.

2. The cosmetic composition as claimed in claim 1, wherein the metal oxide nanopigments have a mean diameter of between 5 and 50 nm.

3. The cosmetic composition as claimed in claim 1 or 2, wherein the metal oxide is titanium oxide.

4. The cosmetic composition as claimed in any one of claims 1 to 3, wherein in the formula (I), Mⁿ⁺ denotes Ca²⁺, Zn²⁺, Mg²⁺ Ba²⁺, Al³⁺ or Zr⁴⁺.

5. The cosmetic composition as claimed in any one of claims 1 to 4, which comprises in a mixture, a titanium oxide nanopigment and the aluminum salt of the compound of formula (I).

6. The cosmetic composition as claimed in any one of claims 1 to 5, wherein the metal oxide nanopigments are uncoated pigments.

7. The cosmetic composition as claimed in any one of claims 1 to 6, wherein the metal oxide nanopigments are coated pigments having undergone one or more surface treatments of a chemical, electronic, mechanicochemical or mechanical nature with compounds chosen from amino acids, beeswax, fatty acids, fatty alcohols, anionic surface-active agents, lecithins, sodium, potassium, zinc, iron or aluminum salts of fatty acids, sodium hexametaphosphate, metal alkoxides, polyethylene, silicones, proteins, alkanolamines, silicon oxides and metal oxides.

8. The cosmetic composition as claimed in claim 7, wherein the coated metal oxide nanopigments are titanium oxide pigments coated with silica, silica and alumina, silica and iron oxide, alumina and silicone, alumina, alumina and aluminum stearate, alumina and aluminum laurate, iron oxide and iron stearate, zinc oxide and zinc stearate, silica and alumina and silicone, silica and alumina and aluminum stearate and silicone, triethanolamine, stearic acid or sodium hexametaphosphate.

9. The cosmetic composition as claimed in claim 7, wherein the coated metal oxide nanopigments are mixtures of silica-coated titanium dioxide and cerium dioxide or alumina, silica and silicone-coated or alumina, silica and glycerin-coated titanium dioxide and zinc dioxide.

10. The cosmetic composition as claimed in any one of claims 1 to 9, which contains 0.5 to 10% by weight, preferably 1 to 7% by weight, relative to the total weight of the composition, of at least one metal oxide nanopigment.

11. The cosmetic composition as claimed in any one of claims 1 to 10, which contains 0.1 to 10% by weight, preferably 0.25% to 5% by weight, calculated on the basis of the acid relative to the total weight of the composition, of partially or completely neutralized 1,4-benzenedi(3-methylidene-10-camphosulfonic) acid of formula (I).

12. The cosmetic composition as claimed in any one of claims 1 to 11, wherein the partially or completely neutralized 1,4-benzenedi(3-methylidene-10-camphosulfonic) acid/metal oxide nanopigment weight ratio is between 0.05 and 5, and preferably between 0.07 and 3.

13. The cosmetic composition as claimed in any one of claims 1 to 12, which is provided in the form of a lotion, thickened lotion, gel, vesicular dispersion, cream, milk, powder, solid stick, foam or spray.

14. The cosmetic composition as claimed in claim 13, which contains, in addition, cosmetic adjuvants chosen from fatty substances, organic solvents, silicones, thickeners, demulcents, UV-A, UV-B or broad-band sun-screen agents, antifoaming agents, moisturizing agents, perfumes, preservatives, surface-active agents, fillers, sequestrants, anionic, cationic, nonionic or amphoteric polymers or mixtures thereof, propellants, alkalizing or acidifying agents, colorants and metal oxide pigments with a particle size of between 100 nm and 20,000 nm.

15. The cosmetic composition as claimed in any one of claims 1 to 14, which contains 3-(4-methylbenzylidene)camphor and 4-tert-butyl-4'-methoxydibenzoylmethane, a titanium oxide nanopigment and 1,4-benzenedi(3-methylidene-10-camphosulfonic) acid which is partially or completely neutralized with triethanolamine.

16. The cosmetic composition as claimed in any one of claims 1 to 15, consisting of a composition for protecting the human epidermis against ultraviolet rays or an anti-sun composition, or for the prevention of photodermatoses, which is provided in the form of a suspension or dispersion in solvents or fatty substances, in the form of an emulsion, in the form of a vesicular dispersion, in the form of an ointment, in the form of a gel, in the form of a solid stick or aerosol foam.

17. The cosmetic composition as claimed in any one of claims 1 to 14 useful for protecting the hair against ultraviolet rays, which is provided in the form of a rinse-off shampoo, lotion, gel or composition to be applied before or after shampooing, before or after dyeing or bleaching, before, during or after permanent waving or hair straightening, in the form of hair styling or treatment lotion or gel, a lotion or gel for blowdrying or hair setting, hair lacquer, composition for permanent waving or hair straightening, for dyeing or bleaching the hair.

18. The cosmetic composition as claimed in any one of claims 1 to 14, consisting of a make-up for the eyelashes, the eyebrows, the skin or the hair, which is provided in the form of a cream for treating the epidermis, foundation, lipstick, eyeshadow, blusher, eyeliner, mascara, coloring gel, and is provided in anhydrous or aqueous solid or pasty form.

19. A process for protecting the human epidermis and the hair against ultraviolet radiation which consists in applying to the skin or the hair, an effective amount of a cosmetic composition as claimed in any one of claims 1 to 18.

20. Use of the cosmetic composition as claimed in any one of claims 1 to 16 or preventing the development of photodermatoses.

21. Use of partially or completely neutralized 1,4-benzenedi(3-methylidene-10-camphosulfonic) acid for reducing or inhibiting the light-induced reaction of metal oxide nanopigments exposed to light, these metal oxides being chosen from titanium, zinc, cerium or zirconium oxides or mixtures thereof with a mean diameter of less than 100 nm, and preferably of between 5 and 50 nm.

## Patentansprüche

1. Filternde kosmetische Zusammensetzung,
dadurch **gekennzeichnet**, daß
sie, in einem kosmetisch verträglichen Trägermedium, mindestens ein Nanopigment aus Metalloxiden, ausgewählt aus den Oxiden von Titan, Zink, Cer, Zirkon oder deren Mischungen, mit einem mittleren Durchmesser unterhalb 100 nm, in Mischung mit teilweise oder vollständig neutralisiertem 1,4-Di(3-methyliden-10-kamphosulfonsäure)benzol der Formel enthält: 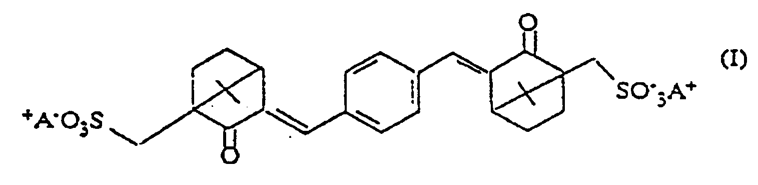 worin A ein Wasserstoffatom, ein Alkalimetall, eine NH(R)₃⁺-Gruppe, deren Reste R gleich oder verschieden sind und ein Wasserstoffatom oder einen C₁₋₄-Alkyl- oder -Hydroxyalkylrest darstellen, oder eine Gruppe Mⁿ⁺/n bedeutet, worin Mⁿ⁺ ein mehrwertiges Metallkation ist, worin n gleich 2 oder 3 oder 4 ist.

2. Kosmetische Zusammensetzung nach Anspruch 1,
dadurch **gekennzeichnet**, daß
die Nanopigmente aus Metalloxiden einen mittleren Durchmesser von 5 bis 50 nm aufweisen.

3. Kosmetische Zusammensetzung gemäß Anspruch 1 oder 2,
dadurch **gekennzeichnet**, daß
das Metalloxid Titanoxid ist.

4. Kosmetische Zusammensetzung gemäß jedem der Ansprüche 1 bis 3,
dadurch **gekennzeichnet**, daß
in Formel (I) Mⁿ⁺ Ca²⁺, Zn²⁺, Mg²⁺, Ba²⁺, Al³⁺ oder Zr⁴⁺ bedeutet.

5. Kosmetische Zusammensetzung gemäß jedem der Ansprüche 1 bis 4,
dadurch **gekennzeichnet**, daß
sie in Mischung ein Nanopigment aus Titanoxid und das Aluminiumsalz der Verbindung der Formel (I) enthält.

6. Kosmetische Zusammensetzung gemäß jedem der Ansprüche 1 bis 5,
dadurch **gekennzeichnet**, daß
die Nanopigmente aus Metalloxiden nicht-umhüllte Pigmente sind.

7. Kosmetische Zusammensetzung gemäß jedem der Ansprüche 1 bis 6,
dadurch **gekennzeichnet**, daß
die Nanopigmente aus Metalloxiden umhüllte Pigmente sind, die einer oder mehreren Oberflächenbehandlungen chemischer, elektronischer, mechanochemischer oder mechanischer Art mit Verbindungen unterzogen worden sind, ausgewählt aus Aminosäuren, Bienenwachs, Fettsäuren, Fettalkoholen, anionischen oberflächenaktiven Mitteln, Lecithinen, Fettsäuresalzen von Natrium, Kalium, Zink, Eisen oder Aluminium, aus Natriumhexametaphosphat, Metallalkoxiden, Polyethylen, Silikonen, Proteinen, Alkanolaminen, Siliziumoxiden sowie aus Metalloxiden.

8. Kosmetische Zusammensetzung gemäß Anspruch 7,
dadurch **gekennzeichnet**, daß
die eingehüllten Nanopigmente aus Metalloxiden Pigmente aus Titanoxiden sind, die umhüllt sind mit Silizium-, Silizium- und Aluminiumoxid, Silizium- und Eisenoxid, Aluminiumoxid und Silikon, Aluminiumoxid, Aluminiumoxid und Aluminiumstearat, Aluminiumoxid und Aluminiumlaurat, Eisenoxid und Eisenstearat, Zinkoxid und Zinkstearat, Silizium- und Aluminiumoxid und Silikon, Silizium- und Aluminiumoxid und Aluminiumstearat und Silikon, Triethanolamin, Stearinsäure oder mit Natriumhexametaphosphat.

9. Kosmetische Zusammensetzung gemäß Anspruch 7,
dadurch **gekennzeichnet**, daß
die umhüllten Nanopigmente aus Metalloxiden Mischungen aus Titandioxid und Cerdioxid sind, welche umhüllt sind mit Silizium- oder Titandioxid und Zinkoxid, mit Aluminiumoxid, Siliziumdioxid und Silikon oder Aluminiumoxid, Siliziumdioxid und Glycerin.

10. Kosmetische Zusammensetzung gemäß jedem der Ansprüche 1 bis 9,
dadurch **gekennzeichnet**, daß
sie 0,5 bis 10 Gew.%, vorzugsweise 1 bis 7 Gew.%, bezogen auf Gesamtgewicht der Zusammensetzung, mindestens eines Nanopigments aus Metalloxid enthält.

11. Kosmetische Zusammensetzung gemäß jedem der Ansprüche 1 bis 10,
dadurch **gekennzeichnet**, daß
sie 0,1 bis 10 Gew.%, vorzugsweise 0,25 bis 5 Gew.%, berechnet auf Basis der Säure, bezogen auf das Gesamtgewicht der Zusammensetzung, an teilweise oder vollständig neutralisiertem 1,4-Di(3-methyliden-10-kamphosulfonsäure)benzol der Formel (I) enthält.

12. Kosmetische Zusammensetzung gemäß jedem der Ansprüche 1 bis 11,
dadurch **gekennzeichnet**, daß
das Gewichtsverhältnis teilweise oder vollständig neutralisiertes 1,4-Di(3-methyliden-10-kamphosulfonsäure)benzol/Nanopigment aus Metalloxid 0,05 bis 5 und vorzugsweise 0,07 bis 3 beträgt.

13. Kosmetische Zusammensetzung gemäß jedem der Ansprüche 1 bis 12,
dadurch **gekennzeichnet**, daß
sie in Form einer Lotion, verdickten Lotion, eines Gels, einer bläschenartigen Dispersion, einer Creme, Milch, eines Pulvers, Feststoffstäbchens, Schaums oder Spray vorliegt.

14. Kosmetische Zusammensetzung gemäß Anspruch 13,
dadurch **gekennzeichnet**, daß
sie ausserdem kosmetische Hilfstoffe enthält, ausgewählt aus Fettkörpern, organischen Lösungsmitteln, Silikonen, Verdickungsmitteln, Weichmachern, Sonnenfilterstoffen für UV-A, UV-B oder für den langwelligen Bandenbereich, aus Antischaummitteln, Hydratisiermitteln, Parfüm-Produkten, Konservierungsstoffen, oberflächenaktiven Mitteln, Chargiermitteln, Sequestriermitteln, anionischen, kationischen, nicht-ionischen, amphoteren Polymeren oder deren Mischungen, Treibmitteln, alkalisch oder sauer machenden Mitteln, Farbstoffen und aus Pigmenten aus Metalloxiden einer Korngröße von 100 bis 20000 nm.

15. Kosmetische Zusammensetzung gemäß jedem der Ansprüche 1 bis 14,
dadurch **gekennzeichnet**, daß
sie 3-(4-Methylbenzyliden)kampfer und 4-t-Butyl-4'-methoxydibenzoylmethan, ein Nanopigment aus Titanoxid und teilweise oder vollständig mit Triethanolamin neutralisiertes 1,4-Di(3-methyliden-10-kamphosulfonsäure)benzol enthält.

16. Kosmetische Zusammensetzung gemäß jedem der Ansprüche 1 bis 15,
die eine Zusammensetzung zum Schutz der menschlichen Epidermis vor ultravioletten Strahlen oder allgemein vor Sonnenstrahlen oder zur Vorbeugung von Fotodermatosen darstellt,
dadurch **gekennzeichnet**, daß
sie in Form einer Suspension oder Dispersion in Lösungsmitteln oder Fettkörpern, in Form einer Emulsion, in Form einer bläschenartigen Dispersion, in Form einer Pommade, in Form eines Gels, in Form eines Feststoffstäbchens oder eines Aerosolschaums vorliegt.

17. Kosmetische Zusammensetzung gemäß der Ansprüche 1 bis 14, die sich zum Schutz der Haare vor ultravioletten Haaren eignet,
dadurch **gekennzeichnet**, daß
sie in Form eines Schampoo, einer Lotion, eines Gels oder einer Zusammensetzung zum Spülen, zur Aufbringung vor oder nach dem Schamponieren, vor oder nach der Färbung oder Entfärbung, vor, während oder nach einer Dauerwelle oder einem Entfrisieren, in Form einer Frisier- oder Behandlungslotion oder eines entsprechenden Gels, einer Lotion oder eines Gels zum Bürsten oder zur Wellengebung, eines Lacks für Haare, einer Zusammensetzung zur Dauerwelle oder zum Entfrisieren, zur Färbung oder Entfärbung der Haare vorliegt.

18. Kosmetische Zusammensetzung gemäß jedem der Ansprüche 1 bis 14, die ein Produkt zum Schminken der Wimpern, Augenbrauen, der Haut oder der Haare darstellt,
dadurch **gekennzeichnet**, daß
sie in Form einer Creme zur Behandlung der Epidermis, einer Teint-Grundlage, eines Lippenstifts, einer Schminke für die Augenlider, Schminke für die Wangen, eines Liniermittels, auch "Eye-Liner" genannt, eines Maskiermittels, Färbegels und in fester oder pasteuser, wasserfreier oder wässriger Form vorliegt.

19. Verfahren zum Schutz der menschlichen Epidermis und der Haare vor ultravioletter Strahlung,
dadurch **gekennzeichnet**, daß
man auf die Haut oder die Haare eine wirksame Menge einer kosmetischen Zusammensetzung gemäß jedem der Ansprüche 1 bis 18 aufträgt.

20. Verwendung der kosmetischen Zusammensetzung gemäß jedem der Ansprüche 1 bis 16 zur Vorbeugung vor dem Auftreten von Fotodermatosen.

21. Verwendung von teilweise oder vollständig neutralisiertem 1,4-Di(3-methyliden-10-kamphosulfonsäure)benzol zur Herabsetzung oder Inhibierung der fotoinduzierten Reaktion von einer Lichteinwirkung ausgesetzten Nanopigmenten aus Metalloxiden, wobei diese Metalloxide aus Oxiden von Titan, Zink, Cer, Zirkon oder deren Mischungen ausgewählt sind, welche einen mittleren Durchmesser unterhalb 100 nm und vorzugsweise von 5 bis 50 nm aufweisen.
